# EUROPEAN PATENT SPECIFICATION

(11) **EP 4 227 676 B1**
(45) Date of publication and mention of the grant of the patent: **02.10.2024**
(21) Application number: 21819915.6
(22) Date of filing: 08.10.2021
(51) Int. Cl.: G01N 27/48, G01N 33/26

(54) **METHOD FOR MEASURING OXIDATION PARAMETERS OF AN ORGANIC SAMPLE**
VERFAHREN ZUR MESSUNG VON OXIDATIONSPARAMETERN EINER ORGANISCHEN PROBE
MÉTHODE DE MESURE DE PARAMÈTRES D'OXYDATION D'UN ÉCHANTILLON ORGANIQUE

(30) Priority: 09.10.2020 ES 202031029
(43) Date of publication of application: 16.08.2023
(73) Proprietor: Bioquochem S.L., 33011 Oviedo (ES)
(72) Inventor: HEVIA SÁNCHEZ, David, 33428 Llanera, Asturias (ES); MUÑOZ CIMADEVILLA, Henar, 33428 Llanera, Asturias (ES); TAMARGO DÍAZ, Sandra, 33428 Llanera, Asturias (ES)
(74) Representative: TRBL Intellectual Property
(86) International application number: PCT/ES2021/070737
(87) International publication number: WO 2022/074280

(56) References cited:
- WO-A1-2019/224410
- CN-A- 102 507 715
- JIRI SOCHOR ET AL: "Electrochemistry as a Tool for Studying Antioxidant Properties", INT. J. ELECTROCHEM. SCI. INTERNATIONAL JOURNAL OF. SCI, 1 January 2013 (2013-01-01), pages 8464 - 8489, XP055662747
- SHABANI EGZONTINA ET AL: "Deep eutectic solvents (DES) as green extraction media for antioxidants electrochemical quantification in extra-virgin olive oils", TALANTA, ELSEVIER, AMSTERDAM, NL, vol. 215, 2 March 2020 (2020-03-02), XP086136940, ISSN: 0039-9140, [retrieved on 20200302], DOI: 10.1016/J.TALANTA.2020.120880
- ALAÑÓN M E ET AL: "Choline chloride derivative-based deep eutectic liquids as novel green alternative solvents for extraction of phenolic compounds from olive leaf", ARABIAN JOURNAL OF CHEMISTRY, ELSEVIER, AMSTERDAM, NL, vol. 13, no. 1, 31 January 2018 (2018-01-31), pages 1685 - 1701, XP086032937, ISSN: 1878-5352, [retrieved on 20180131], DOI: 10.1016/J.ARABJC.2018.01.003
- MENEZES PEIXOTO CARLOS ROBERTO DE ET AL: "Voltammetric determination of total antioxidant capacity ofBunchosia glanduliferatree extracts", JOURNAL OF ELECTROANALYTICAL CHEMISTRY, ELSEVIER, AMSTERDAM, NL, vol. 799, 4 July 2017 (2017-07-04), pages 519 - 524, XP085195875, ISSN: 1572-6657, DOI: 10.1016/J.JELECHEM.2017.07.003
- PISOSCHI A M ET AL: "Electrochemical Methods for Total Antioxidant Capacity and its Main Contributors Determination: A review", OPEN CHEM,, vol. 13, no. 1, 30 April 2015 (2015-04-30), pages 824 - 856, XP002763100, ISSN: 2391-5420, DOI: 10.1515/CHEM-2015-0099

## Description

### FIELD OF THE INVENTION

The present patent is comprised among analytical methods for measuring some oxidation parameters, such as for example antioxidant and pro-oxidant capacity, oxidation stability, and oxidation resistance by means of electrochemistry techniques in organic samples.

### BACKGROUND OF THE INVENTION

The measurement of some oxidation parameters in organic samples can offer very valuable information about the worth or the degradation state of said samples. Parameters such as antioxidant and pro-oxidant capacity, oxidation resistance under certain conditions, or the stability of said oxidation allow the user to discriminate those samples that fail to meet a series of basic requirements depending on the application in which they are going to be used. These measurements are simple in an aqueous mixture, but the methodology in an organic sample is much more complex.

Some methods intended for measuring the oxidation of an organic sample are known. Rancimat or PetrOxy are methods for measuring the oxidative stability of oils and fats based on the induction of oxidation of the sample by exposure to high temperatures and an air flow. This allows estimating the time of induction or time of oxidative stability, with this being the time after which the sample has exceeded the time during which it remains stable, and therefore being indicative of a loss of quality and service life of the sample. However, it is an indirect measurement, since only the volatile products generated (after applying temperature and air flow for several hours) are quantified, and furthermore the oxidation resistance and antioxidant capacity of the sample at time zero is not directly assessed. It is necessary to perform tests that last a considerable amount of time, for example in the case of Rancimat, for biodiesel samples the mandatory minimum testing time amounts to 6 hours, the testing lasting for a longer time in most cases.

Therefore, to perform both methods a lot of time is needed, and furthermore, the measurement that is taken is an indirect measurement, so it is not a completely reliable method.

Document WO 2019/224410 A1 discloses an apparatus for measuring antioxidant properties of some particular examples. However, this apparatus and method cannot be applied to organic samples, such as oils.

Paper of SHABANI EGZONTINA et al, titled "Deep eutectic solvents (DES) as green extraction media for antioxidants electrochemical quantification in extra-virgin olive oils" discloses the use of DES to prepare samples in a measurement of some antioxidant properties of olive oils. However, it uses a very complex apparatus and method to do so.

The present invention intends to provide an alternative solution to this problem.

### BRIEF DESCRIPTION OF THE INVENTION

The present invention provides an alternative solution to the problem proposed above by means of a method for measuring oxidation parameters according to claim 1. The dependent claims define preferred embodiments of the invention.

Unless defined otherwise, all the terms (both scientific and technical terms) used herein must be interpreted as one skilled in the art would interpret them. It is therefore understood that common terms must be interpreted as one who is familiar with the subject matter would interpret them, and not in an idealized or strictly formal manner.

Throughout the text, the word "comprises" (and its derivatives such as "comprising") should not be understood in an exclusive manner, but rather should be understood to mean that they allow for the possibility that what is defined may include additional elements or steps.

An object of the present invention relates, although without limitation, to a method for measuring oxidation parameters of an organic sample, the method comprising the steps of:
preparing a first compound comprising a natural deep eutectic solvent;
stirring the first compound for a time comprised between 30 and 120 minutes at a temperature comprised between 0 and 100°C and leaving to cool at room temperature, obtaining a first solution
adding water and an inorganic salt to the first solution, obtaining a second solution, wherein the volume of added water represents between 1 % and 40% of the second solution and the inorganic salt is at a concentration comprised between 0.001 M and 1 M in the second solution;
adding at least one additional catalyst compound to the second solution, obtaining a final solution
dissolving the organic sample with the final solution by stirring, giving rise to a measurement mixture;
placing the stirred measurement mixture in an electrochemical transducer containing a working electrode, a reference or pseudo-reference electrode, and an auxiliary electrode;
applying by means of a potentiostat at least one voltage range to the working electrode according to linear sweep voltammetry, with the voltage range being comprised within the interval between 0 and 2 V;
obtaining a current variation signal across said voltage range;
calculating the load for the voltage range applied by the potentiostat by means of the integration of the current variation signal;
transforming loads into adimensional values for obtaining a representative reference value of the antioxidant and/or pro-oxidant capacity of the organic sample.

As a result of this method, an electrochemical measurement device can be used to evaluate the antioxidant capacity of an oily sample, simply by means of the detailed preparation of said sample. A direct and much faster measurement of oxidation of the organic sample is obtained by means of this method.

In fact, this method is valid for measuring several oxidation parameters of the organic sample. Because the method is based on preparing a stable mixture and mixing it with the sample to be measured, successive variants of the method can be used to measure different parameters: using a single measurement the antioxidant or pro-oxidant capacity of the sample is observed; if several measurements are taken without varying the sample, the method can measure the oxidative stability thereof; if several measurements are taken applying heat to the sample, the method can measure oxidation resistance.

Natural deep eutectic solvent (NADES) is understood to be a mixture of two or more chemical compounds at a particular molar ratio to give rise to a significant drop in the melting temperature of the individual components, becoming liquid at room temperature. In this sense, the preparation of the solvents does not require any chemical reaction, and therefore the production yield is 100%.

When mentioning a catalyst compound, it does not mean a catalyst in the strict sense, but rather a compound that facilitates the dissolution and/or improves the reproducibility of the measurement in the first solution.

The voltage range can be applied in both in an increasing sense (where the antioxidant capacity of the sample is intended to be measured) and in a decreasing sense (where the pro-oxidant capacity of the sample is intended to be measured).

By means of adding an inorganic salt, in a combination compensated with the addition of water, conductivity in all the samples is homogenized, regardless of their more or less conductive nature. In this way, reproducibility of the measurements is improved, and a more reliable final measurement is obtained.

In particular embodiments, before placing the measurement mixture in the electrochemical transducer the aqueous phase of the measurement mixture is obtained, and it is the aqueous phase that is placed in the electrochemical transducer.

Separating or obtaining the aqueous phase is especially advantageous when the measurement mixture cannot be directly subjected to the measurements, either because it is too viscous or else because it does not have the properties needed for the measurement to be taken.

In particular embodiments, the step of mixing the organic sample with the final solution further comprises leaving the measurement mixture to stand before obtaining the aqueous phase.

The step of leaving the mixture to stand allows the separation of the aqueous part, which will be analyzed in the apparatus by means of linear sweep voltammetry.

In particular embodiments, the steps of mixing by stirring the organic sample with the final solution, obtaining the aqueous phase of the measurement mixture, placing the stirred aqueous phase in a receiver, and measuring oxidation of the aqueous phase are repeated, after a certain time has lapsed using as an organic sample a second organic sample similar to the first organic sample in which temperature has been applied and air added.

The method of the invention can thereby also be used according to standard UNE 14112 for measuring oxidation stability in certain organic samples.

In particular embodiments, the electrochemical transducer is a fungible strip and comprises a carbon working electrode, a silver pseudo-reference electrode, and a carbon auxiliary electrode.

The method of the invention prepares a measurement mixture which can be analyzed by a portable device by depositing the mixture on a fungible strip. In this manner, the portable device would be in charge of the steps of applying linear voltammetry to the sample and of calculating the antioxidant or pro-oxidant measurement.

In particular embodiments, the step of applying a voltage range to the working electrode is performed linearly, starting with the lowest value in the voltage range and increasing the voltage up to the highest value in the voltage range.

In these cases, the voltage applied follows a linear rule relative to time, governed by a constant comprised between 0.1 mV/s and 500 mV/s.

In particular embodiments, the step of applying a voltage range is performed inversely, starting with the highest value of voltage and decreasing to the lowest value.

The pro-oxidant capacity of the organic sample can thereby be calculated.

In particular embodiments, the step of preparing the first compound is performed by mixing at least two hydrogen bond-forming substances, particularly by preparing one of the following mixtures
choline chloride and lactic acid, wherein the molarity ratio of choline chloride to lactic acid is comprised between 1:1 and 1:4, particularly between 1:1.9 and 1:2.1, or else
choline chloride and glucose, wherein the molarity ratio of choline chloride to glucose is comprised between 1:0.1 and 1:4, particularly between 1:0.9 and 1:1.1, or else
lactic acid and glucose, wherein the molarity ratio of lactic acid to glucose is comprised between 6:0.1 and 6:4, particularly between 6:0.9 and 6:1.1.

In the first case, per mole of choline chloride, between 1 and 4 moles of lactic acid are mixed, particularly between 1.9 and 2.1 moles of lactic acid. In the second case, per mole of choline chloride, between 0.1 and 4 moles of glucose are mixed, particularly between 0.9 and 1.1 moles of glucose. In the third case, per 6 moles of lactic acid, between 0.1 and 4 moles of glucose are mixed, particularly between 0.9 and 1.1 moles of glucose.

While these ratios are not the only options, they have allowed a natural deep eutectic solvent to be obtained in a simple and reliable manner.

In particular embodiments, the step of mixing the organic sample with the final solution is performed at a temperature comprised between 0°C and 60°C, particularly between 30°C and 40°C, stirring the mixture.

Though not essential, the range is one that is beneficial for keeping the properties of the organic sample to be analyzed intact. Stirring allows for better homogenization of the mixture, such that the results show less dispersion.

In particular embodiments, the steps of placing the mixture in a receiver, applying a voltage range, obtaining a signal, calculating the load, and transforming loads into adimensional values are performed more than once, stirring the mixture before performing each of the steps.

The mean of the different measurements can thereby be calculated, obtaining a value that is less affected by the possible variability due to the lack of complete homogeneity of the sample to be analyzed.

In particular embodiments, once the first solution is obtained, it is kept at room temperature in a container in the dark.

The absence of light is favorable for maintaining the properties of the mixture, preventing the light from degrading certain components.

In particular embodiments, the first solution is mixed with an organic solvent before adding the catalyst compound, wherein the ratio by volume between the second solution and the organic solvent is comprised between 2:1 and 1:5, particularly comprised between 1:0.9 and 1:1.1.

This organic solvent, such as for example, a mixture of hexane and acetone at volumetric ratio of 1:1, allows improving the extraction.

In particular embodiments, the catalyst compound is an organic salt, such as for example tetrabutylammonium hexafluorophosphate, or TBAPF6, at a final concentration comprised between 0.1 and 5 mM, particularly between 0.9 and 1.1 mM.

As a result of adding this salt, the conductivity and solubility in non-aqueous solutions is improved.

In particular embodiments, the step of mixing the organic sample with the final solution is carried out with a volumetric ratio between the solution and the sample comprised between 24:1 and 0.5:1, especially between 1:0.95 and 1:1.05.

This means that per liter of organic sample, between 1.2 and 24 liters of the final solution obtained in the steps of the method according to the invention are mixed. This wide range of volumetric ratios is suitable for correctly measuring the antioxidant or pro-oxidant capacity of the sample. The particular range comprised between 1:0.95 and 1:1.05 works especially well with oil samples.

In particular embodiments, the catalyst compound is a strong organic acid, such as for example methanesulfonic acid, wherein the ratio by volume between the second solution and the catalyst is comprised between 4:0.25 and 4:3, particularly comprised between 4:0.95 and 4:1.05.

Adding methanesulfonic acid improves the extraction of compounds soluble in aqueous phase.

In particular embodiments, the step of mixing the organic sample with the final solution is carried out with a volumetric ratio between the solution and the sample comprised between 4:0.25 and 1.25:1, especially between 4:0.95 and 4:1.05. The particular range comprised between 4:0.95 and 4:1.05 works especially well with biodiesel samples.

### DESCRIPTION OF THE FIGURES

A brief description of each of the figures used to complete the following description of the invention is provided. Said figures are related to the state of the art or to preferred embodiments of the invention, presented as non-limiting examples thereof.
Figure 1 shows the steps of a first method according to the invention.
Figure 2 shows the steps of a second method according to the invention.

### DETAILED DESCRIPTION OF THE INVENTION

An example of preferred embodiment of the present invention, provided for illustrative but non-limiting purposes, is described below.

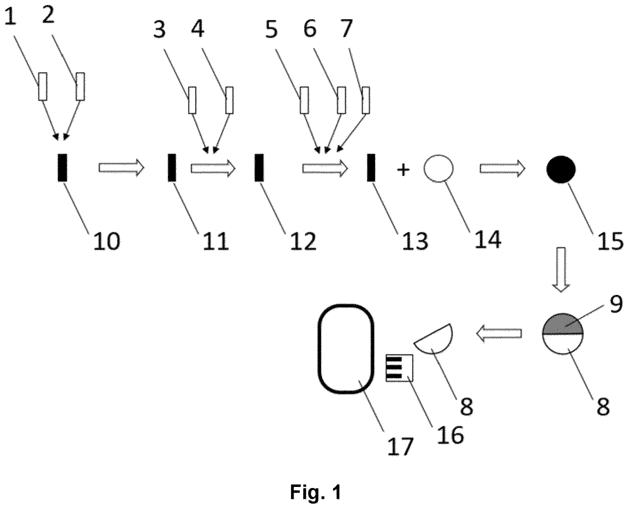

Figure 1 shows the steps of a first method according to the invention. This first method is especially suited for measuring the antioxidant or pro-oxidant capacity of an oil sample.

In a first step, this method includes the preparation of an extraction and measurement solution 10. This extraction and measurement solution 10 is prepared by means of the mixture of two compounds, giving rise to a natural deep eutectic solvent, also known as NADES.

This natural deep eutectic solvent is formed from a hydrogen bond donor and a hydrogen bond acceptor, In this method in particular, it is obtained by mixing choline chloride 1 and lactic acid 2, wherein the molarity ratio of choline chloride to lactic acid is 1:2.

This extraction and measurement solution is stirred at a temperature of 50°C for 75 minutes and left to cool until reaching room temperature, obtaining what is referred to as the first solution 11.

In a second step, water 3, together with an inorganic salt, 4 such as for example KCI, is added to the first solution 11. A second solution 12 is thereby obtained. The percentage of water added represents 20% of the volume of the second solution 12, and the final concentration of KCI in the second solution 12 is 0.05 M. These components are mixed until the second solution is homogeneous. Once this homogeneous solution is obtained, it is kept at room temperature in a container in the dark.

This second solution is mixed with acetone 5 and hexane 6, in a volumetric ratio of 2:1:1, and then an organic salt 7, such as for example tetrabutylammonium hexafluorophosphate, or TBAPF6, at a final concentration of 1 mM, is added. A final solution 13 is thereby obtained. This final solution is independent of the sample to be analyzed, so it can be prepared prior to being mixed with the sample the oxidation of which is to be measured. It is even possible to prepare the final solution and provide it separately, decisively contributing to carrying out the method according to the invention.

Once this final solution 13 is obtained, it is mixed with the organic sample 14 the antioxidant or pro-oxidant capacity of which is to be measured. In this case, the ratio used in this mixture is 1 volume of final solution 13 per volume of organic sample 14. The step of mixing the organic sample with the final solution is performed at a temperature comprised between 0°C and 60°C.

This mixture 15 is stirred in the vortex for 30 seconds and left to stand for 15 minutes at room temperature until the aqueous phase 8 separates from the oily phase 9.

This separation of phases is only necessary in some examples, such as in this case, where the characteristics of the oily phase make it difficult to measure the load. In other examples, it is possible to continue without a separation of phases, directly placing the mixture 15 on the fungible strip, according to the method described below.

The aqueous phase 8 is separated and stirred to homogenize same, and the antioxidant capacity of said aqueous phase is then measured in triplicate, stirring the sample before each measurement.

The antioxidant capacity is measured as follows (these steps are the steps that are performed in triplicate, then obtaining the mean value thereof):
placing the stirred aqueous phase on a fungible strip 16 containing a carbon working electrode, an Ag pseudo-reference electrode, and a carbon auxiliary electrode;
applying by means of a potentiostat 17 at least one voltage range to the working electrode according to linear sweep voltammetry, with the voltage range being comprised within the interval between 0 and 2 V;
obtaining a current variation signal across said voltage range;
calculating the load for the voltage range applied by the potentiostat by means of the integration of the current variation signal,
transforming loads into adimensional values for obtaining a representative reference value of the antioxidant and/or pro-oxidant capacity of the organic sample.

Conversely, if the pro-oxidant capacity of the sample is to be measured, the voltage range will be applied inversely, i.e., from 2 V to 0 V.

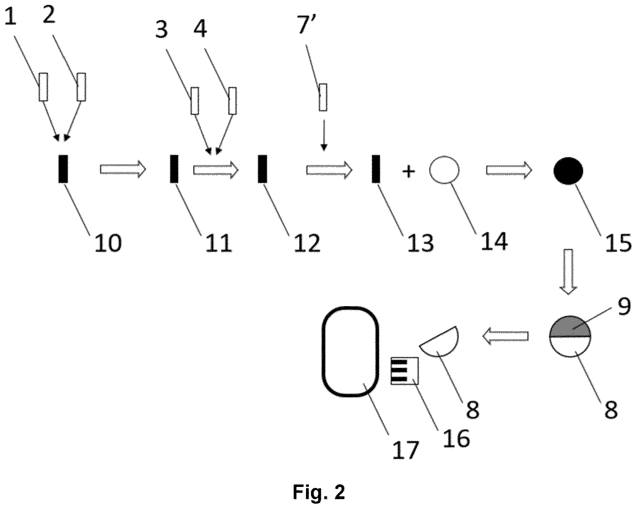

Figure 2 shows the steps of a second method according to the invention. This second method is especially suited for measuring the antioxidant or pro-oxidant capacity of a biodiesel sample.

The first step is performed in an identical manner relative to the first step of the above method, obtaining a first solution 11 with a natural deep eutectic solvent.

The second step is also identical to the second step of the above method, obtaining a homogeneous second solution 12.

In this particular method, a strong organic acid 7', such as for example methanesulfonic acid, is added to this second solution, wherein the ratio by volume between the second solution 12 and the strong organic acid 7' is 4:1. A final solution 13 is thereby obtained. This final solution is independent of the sample to be analyzed, so it can be prepared prior to being mixed with the sample the oxidation of which is to be measured. It is even possible to prepare the final solution and provide it separately, decisively contributing to carrying out the method according to the invention.

Once this final solution 13 is obtained, it is mixed with the organic sample 14 the antioxidant or pro-oxidant capacity of which is to be measured. In this case, the ratio used in this mixture is 4 volumes of final solution 13 per volume of organic sample 14. The step of mixing the organic sample with the final solution is performed at a temperature of 40°C, stirring the mixture 15 for 5 minutes and allowing said mixture to stand for 15 minutes at room temperature until the aqueous phase 8 separates from the oily phase 9.

The aqueous phase 8 is separated and stirred to homogenize same, and the antioxidant capacity of said aqueous phase is then measured in triplicate, stirring the sample before each measurement.

The antioxidant capacity is measured as follows (these steps are the steps that are performed in triplicate, then obtaining the mean value thereof):
placing the stirred aqueous phase on a fungible strip 16 containing a carbon working electrode, an Ag pseudo-reference electrode, and a carbon auxiliary electrode;
applying by means of a potentiostat 17 at least one voltage range to the working electrode according to linear sweep voltammetry, with the voltage range being comprised within the interval between 0 and 2 V
obtaining a current variation signal across said voltage range;
calculating the load for the voltage range applied by the potentiostat by means of the integration of the current variation signal,
transforming loads into adimensional values for obtaining a representative reference value of the antioxidant and/or pro-oxidant capacity of the organic sample.

Conversely, if the pro-oxidant capacity of the sample is to be measured, the voltage range will be applied inversely, i.e., from 2 V to 0 V.

## Claims

1. A method for measuring oxidation parameters of an organic sample, the method comprising the steps of:
preparing a first compound (10) comprising a natural deep eutectic solvent;
stirring the first compound (10) for a time comprised between 30 and 120 minutes at a temperature comprised between 0 and 100°C and leaving to cool at room temperature, obtaining a first solution (11),
adding water (3) and an inorganic salt (4) to the first solution, obtaining a second solution (12), wherein the volume of added water represents between 1% and 40% of the second solution (12), and the inorganic salt (4) is at a concentration comprised between 0.001 M and 1 M in the second solution (12)
adding at least one additional catalyst compound (7, 7') to the second solution (12), obtaining a final solution (13), wherein the catalyst compound is a strong organic acid, such as for example the methanesulfonic acid, wherein the ratio by volume between the second solution and the catalyst is comprised between 4:0.25 and 4:3, particularly comprised between 4:0.95 and 4:1.05;
dissolving the organic sample (14) with the final solution (13) by stirring, giving rise to a measurement mixture (15);
placing the stirred measurement mixture in a fungible strip (16) containing a carbon working electrode, a silver pseudo-reference electrode, and a carbon auxiliary electrode;
applying by means of a potentiostat (17) at least one voltage range to the working electrode according to linear sweep voltammetry, with the voltage range being comprised within the interval between 0 and 2 V;
obtaining a current variation signal across said voltage range;
calculating the load for the voltage range applied by the potentiostat by means of the integration of the current variation signal;
transforming loads into adimensional values for obtaining a representative reference value of the antioxidant and/or pro-oxidant capacity of the organic sample.

2. The method according to claim 1, wherein before placing the measurement mixture in the electrochemical transducer the aqueous phase of the measurement mixture is obtained, and it is the aqueous phase that is placed in the electrochemical transducer.

3. The method according to claim 2, wherein the step of mixing the organic sample (14) with the final solution (13) further comprises leaving the measurement mixture (15) to stand before obtaining the aqueous phase (8).

4. The method according to any of claims 2 or 3, wherein the steps of mixing by stirring the organic sample with the final solution, obtaining the aqueous phase (8) of the measurement mixture (15), placing the stirred aqueous phase in a receiver, and measuring oxidation of the aqueous phase, are repeated, after a certain time has lapsed using as an organic sample a second organic sample similar to the first organic sample to which the addition of air and temperature have been applied.

5. The method according to any of the preceding claims, wherein the step of applying a voltage range to the working electrode is performed linearly, starting with the lowest value in the voltage range and increasing the voltage up to the highest value in the voltage range.

6. The method according to any of claims 1 to 4, wherein the step of applying a voltage range is performed inversely, starting with the highest value of voltage and decreasing to the lowest value.

7. The method according to any of the preceding claims, wherein the step of preparing the first compound is performed by mixing at least two hydrogen bond-forming substances, particularly by preparing one of the following mixtures
choline chloride (1) and lactic acid (2), wherein the molarity ratio of choline chloride (1) to lactic acid (2) is comprised between 1:1 and 1:4, particularly between 1:1.9 and 1:2.1, or else
choline chloride and glucose, wherein the molarity ratio of choline chloride to glucose is comprised between 1:0.1 and 1:4, particularly between 1:0.9 and 1:1.1, or else
lactic acid and glucose, wherein the molarity ratio of lactic acid to glucose is comprised between 6:0.1 and 6:4, particularly between 6:0.9 and 6:1.1.

8. The method according to any of the preceding claims, wherein the step of mixing the organic sample (14) with the final solution (13) is performed at a temperature comprised between 0°C and 60°C, particularly between 30°C and 40°C, stirring the mixture.

9. The method according to any of the preceding claims, wherein the steps of placing the mixture in a receiver, applying a voltage range, obtaining a signal, calculating the load, and transforming loads into adimensional values are performed more than once, stirring the mixture before performing each of the steps.

10. The method according to any of the preceding claims, wherein once the first solution is obtained, it is kept at room temperature in a container in the dark.

11. The method according to claim 10, where the first solution (11) is mixed with an organic solvent (5, 6) before adding the catalyst compound (7, 7'), wherein the ratio by volume between the second solution and the organic solvent is comprised between 2:1 and 1:5, particularly comprised between 1:0.9 and 1:1.1.

12. The method according to any of claims 10 or 11, wherein the catalyst compound is an organic salt, such as for example the tetrabutylammonium hexafluorophosphate, or TBAPF6, at a final concentration comprised between 0.1 and 5 mM, particularly between 0.9 and 1.1 mM.

13. The method according to any of claims 10 to 12, wherein the step of mixing the organic sample with the final solution is carried out with a volumetric ratio between the solution and the sample comprised between 24:1 and 0.5:1, especially between 1:0.95 and 1:1.05.

14. The method according to any of the preceding claims, wherein the step of mixing the organic sample with the final solution is carried out with a volumetric ratio between the final solution and the sample comprised between 20:1 and 1.25:1, especially between 4:0.95 and 4:1.05.

## Patentansprüche

1. Verfahren zur Messung von Oxidationsparametern einer organischen Probe, wobei das Verfahren die folgenden Schritte umfasst:
Herstellen einer ersten Verbindung (10), die ein natürliches stark eutektisches Lösungsmittel enthält;
Rühren der ersten Verbindung (10) über einen Zeitraum von 30 bis 120 Minuten bei einer Temperatur zwischen 0 und 100 °C und Abkühlenlassen bei Raumtemperatur, wodurch sich eine erste Lösung (11) ergibt,
Zugabe von Wasser (3) und eines anorganischen Salzes (4) zu der ersten Lösung, wodurch sich eine zweite Lösung (12) ergibt, wobei das Volumen des zugegebenen Wassers zwischen 1 % und 40 % der zweiten Lösung (12) ausmacht und die Konzentration des anorganischen Salzes (4) in der zweiten Lösung (12) zwischen 0,001 M und 1 M liegt
Zugabe von mindestens einer zusätzlichen Katalysatorverbindung (7, 7') zu der zweiten Lösung (12), wodurch sich eine endgültige Lösung (13) ergibt, wobei die Katalysatorverbindung eine starke organische Säure wie z. B. Methansulfonsäure ist, wobei das Volumenverhältnis der zweiten Lösung zum Katalysator zwischen 4:0,25 und 4:3, insbesondere zwischen 4:0,95 und 4:1,05, liegt;
Auflösen der organischen Probe (14) durch Rühren zusammen mit der endgültigen Lösung (13), sodass ein Messgemisch (15) entsteht;
Aufbringen des verrührten Messgemischs auf einen austauschbaren Streifen (16), der eine Kohlenstoff-Arbeitselektrode, eine Silber-Pseudoreferenzelektrode und eine Kohlenstoff-Hilfselektrode enthält;
Anlegen mindestens eines Spannungsbereichs an die Arbeitselektrode mittels eines Potentiostaten (17) nach dem Verfahren der Linear-Sweep-Voltammetrie, wobei der Spannungsbereich im Intervall zwischen 0 und 2 V liegt;
Ermitteln eines Stromschwankungssignals über den genannten Spannungsbereich;
Berechnen der Last für den vom Potentiostaten angelegten Spannungsbereich durch Einbeziehung des Stromschwankungssignals;
Umwandeln der Lasten in dimensionslose Werte zur Ermittlung eines repräsentativen Referenzwerts für die antioxidative und/oder prooxidative Kapazität der organischen Probe.

2. Verfahren nach Anspruch 1, wobei vor Einbringen des Gemischs in den elektrochemischen Aufnehmer die wässrige Phase des Gemischs gewonnen und dann diese wässrige Phase in den elektrochemischen Aufnehmer eingebracht wird.

3. Verfahren nach Anspruch 2, wobei der Schritt des Mischens der organischen Probe (14) mit der endgültigen Lösung (13) außerdem das Stehenlassen des Messgemischs (15) vor Gewinnung der wässrigen Phase (8) umfasst.

4. Verfahren nach einem der Ansprüche 2 oder 3, wobei die Schritte des Mischens der organischen Probe mit der endgültigen Lösung durch Rühren, des Gewinnens der wässrigen Phase (8) des Messgemischs (15), des Einbringens der verrührten wässrigen Phase in eine Aufnahmevorrichtung und des Messens der Oxidation der wässrigen Phase nach Ablauf einer bestimmten Zeit wiederholt werden, wobei als organische Probe eine der ersten organischen Probe ähnliche zweite organische Probe unter Zufuhr von Luft und Temperatur verwendet wird.

5. Verfahren nach einem der vorstehenden Ansprüche, wobei der Schritt des Anlegens eines Spannungsbereichs an die Arbeitselektrode linear - beginnend mit dem niedrigsten Wert im Spannungsbereich und Steigerung der Spannung bis zum höchsten Wert im Spannungsbereich - erfolgt.

6. Verfahren nach einem der Ansprüche 1 bis 4, wobei der Schritt des Anlegens eines Spannungsbereichs umgekehrt - beginnend mit dem höchsten Wert der Spannung und abnehmend bis zum niedrigsten Wert - erfolgt.

7. Verfahren nach einem der vorstehenden Ansprüche, wobei der Schritt des Herstellens der ersten Verbindung durch Mischen von mindestens zwei wasserstoffbrückenbildenden Stoffen, insbesondere durch Herstellen eines der folgenden Gemische, erfolgt:
Cholinchlorid (1) und Milchsäure (2), wobei das Stoffmengenverhältnis von Cholinchlorid (1) zu Milchsäure (2) zwischen 1:1 und 1:4, insbesondere zwischen 1:1,9 und 1:2,1, liegt, oder aber
Cholinchlorid und Glukose, wobei das Stoffmengenverhältnis von Cholinchlorid zu Glukose zwischen 1:0,1 und 1:4, insbesondere zwischen 1:0,9 und 1:1,1, liegt, oder aber
Milchsäure und Glukose, wobei das Molverhältnis von Milchsäure zu Glukose zwischen 6:0,1 und 6:4, insbesondere zwischen 6:0,9 und 6:1,1, liegt.

8. Verfahren nach einem der vorstehenden Ansprüche, wobei der Schritt des Mischens der organischen Probe (14) mit der endgültigen Lösung (13) unter Rühren des Gemischs bei einer Temperatur zwischen 0 °C und 60 °C, insbesondere zwischen 30 °C und 40 °C, erfolgt.

9. Verfahren nach einem der vorstehenden Ansprüche, wobei die Schritte des Einbringens des Gemischs in eine Aufnahmevorrichtung, des Anlegens eines Spannungsbereichs, des Ermittelns eines Signals, des Berechnens der Last und des Umwandelns von Lasten in dimensionslose Werte mehr als einmal durchgeführt werden, wobei das Gemisch vor Durchführung der einzelnen Schritte gerührt wird.

10. Verfahren nach einem der vorstehenden Ansprüche, wobei die erste Lösung nach ihrer Herstellung in einem Behälter bei Raumtemperatur und im Dunkeln aufbewahrt wird.

11. Verfahren nach Anspruch 10, wobei die erste Lösung (11) vor der Zugabe der Katalysatorverbindung (7, 7') mit einem organischen Lösungsmittel (5, 6) gemischt wird, wobei das Volumenverhältnis der zweiten Lösung zum organischen Lösungsmittel zwischen 2:1 und 1:5, insbesondere zwischen 1:0,9 und 1:1,1, liegt.

12. Verfahren nach einem der Ansprüche 10 oder 11, wobei die Katalysatorverbindung ein organisches Salz wie beispielsweise Tetrabutylammoniumhexafluorophosphat (TBAPF6) mit einer Endkonzentration zwischen 0,1 und 5 mM, insbesondere zwischen 0,9 und 1,1 mM, ist.

13. Verfahren nach einem der Ansprüche 10 bis 12, wobei der Schritt des Mischens der organischen Probe mit der endgültigen Lösung in einem Volumenverhältnis der Lösung zur Probe zwischen 24:1 und 0,5:1, insbesondere zwischen 1:0,95 und 1:1,05, erfolgt.

14. Verfahren nach einem der vorstehenden Ansprüche, wobei der Schritt des Mischens der organischen Probe mit der endgültigen Lösung in einem Volumenverhältnis der endgültigen Lösung zur Probe zwischen 20:1 und 1,25:1, insbesondere zwischen 4:0,95 und 4:1,05 erfolgt.

## Revendications

1. Méthode de mesure de paramètres d'oxydation d'un échantillon organique, la méthode comprenant les étapes suivantes :
préparer un premier composé (10) comprenant un solvant eutectique profond naturel ;
agiter le premier composé (10) pendant une durée comprise entre 30 et 120 minutes à une température comprise entre 0 et 100°C et laisser refroidir à température ambiante, pour obtenir une première solution (11),
ajouter de l'eau (3) et un sel inorganique (4) à la première solution, pour obtenir une deuxième solution (12), dans laquelle le volume d'eau ajouté représente entre 1 % et 40 % de la deuxième solution (12), et le sel inorganique (4) est à une concentration comprise entre 0,001 M et 1 M dans la deuxième solution (12),
ajouter au moins un composé catalytique supplémentaire (7, 7') à la deuxième solution (12), pour obtenir une solution finale (13), dans laquelle le composé catalytique est un acide organique fort, comme par exemple l'acide méthanesulfonique, dans laquelle le rapport en volume entre la deuxième solution et le catalyseur est compris entre 4:0,25 et 4:3, en particulier compris entre 4:0,95 et 4:1,05 ;
dissoudre l'échantillon organique (14) dans la solution finale (13) en agitant, pour donner lieu à un mélange de mesure (15) ;
placer le mélange de mesure agité dans une bande fongible (16) contenant une électrode de travail au carbone, une électrode de pseudo-référence à l'argent et une électrode auxiliaire au carbone ;
appliquer au moyen d'un potentiostat (17) au moins une plage de tension à l'électrode de travail selon la voltamétrie à balayage linéaire, la plage de tension étant comprise dans l'intervalle entre 0 et 2 V ;
obtenir un signal de variation du courant dans ladite plage de tension ;
calculer la charge pour la plage de tension appliquée par le potentiostat au moyen de l'intégration du signal de variation du courant ;
transformer les charges en valeurs adimensionnelles pour obtenir une valeur de référence représentative de la capacité antioxydante et/ou pro-oxydante de l'échantillon organique.

2. Méthode selon la revendication 1, dans laquelle, avant de placer le mélange de mesure dans le transducteur électrochimique, on obtient la phase aqueuse du mélange de mesure, et c'est la phase aqueuse qui est placée dans le transducteur électrochimique.

3. Méthode selon la revendication 2, dans laquelle l'étape consistant à mélanger l'échantillon organique (14) avec la solution finale (13) comprend en outre laisser reposer le mélange de mesure (15) avant d'obtenir la phase aqueuse (8).

4. Méthode selon l'une quelconque des revendications 2 ou 3, dans laquelle les étapes consistant à mélanger en agitant l'échantillon organique avec la solution finale, à obtenir la phase aqueuse (8) du mélange de mesure (15), à placer la phase aqueuse agitée dans un récepteur et à mesurer l'oxydation de la phase aqueuse, sont répétées, après un certain temps, en utilisant comme échantillon organique un deuxième échantillon organique similaire au premier échantillon organique auquel l'ajout d'air et de température a été appliqué.

5. Méthode selon l'une quelconque des revendications précédentes, dans laquelle l'étape consistant à appliquer une plage de tension à l'électrode de travail est réalisée de manière linéaire, en commençant par la valeur la plus faible de la plage de tension et en augmentant la tension jusqu'à la valeur la plus élevée de la plage de tension.

6. Méthode selon l'une quelconque des revendications 1 à 4, dans laquelle l'étape consistant à appliquer une plage de tension est effectuée inversement, en commençant par la valeur de tension la plus élevée et en diminuant jusqu'à la valeur la plus faible.

7. Méthode selon l'une quelconque des revendications précédentes, dans laquelle l'étape consistant à préparer le premier composé est réalisée en mélangeant au moins deux substances formant des liaisons hydrogène, notamment en préparant l'un des mélanges suivants
du chlorure de choline (1) et de l'acide lactique (2), dans laquelle le rapport molaire du chlorure de choline (1) par rapport à l'acide lactique (2) est compris entre 1:1 et 1:4, en particulier entre 1:1,9 et 1:2,1, ou encore
du chlorure de choline et du glucose, dans laquelle le rapport molaire du chlorure de choline par rapport au glucose est compris entre 1:0,1 et 1:4, en particulier entre 1:0,9 et 1:1,1, ou encore
de l'acide lactique et du glucose, dans laquelle le rapport molaire de l'acide lactique par rapport au glucose est compris entre 6:0,1 et 6:4, en particulier entre 6:0,9 et 6:1,1.

8. Méthode selon l'une quelconque des revendications précédentes, dans laquelle l'étape consistant à mélanger l'échantillon organique (14) avec la solution finale (13) est effectuée à une température comprise entre 0°C et 60°C, en particulier entre 30°C et 40°C, en agitant le mélange.

9. Méthode selon l'une quelconque des revendications précédentes, dans laquelle les étapes consistant à placer le mélange dans un récepteur, à appliquer une plage de tension, à obtenir un signal, à calculer la charge et à transformer les charges en valeurs adimensionnelles sont effectuées plus d'une fois, en agitant le mélange avant d'effectuer chacune des étapes.

10. Méthode selon l'une quelconque des revendications précédentes, dans laquelle, une fois la première solution obtenue, elle est conservée à température ambiante dans un récipient à l'abri de la lumière.

11. Méthode selon la revendication 10, dans laquelle la première solution (11) est mélangée avec un solvant organique (5, 6) avant d'ajouter le composé catalytique (7, 7'), dans laquelle le rapport en volume entre la deuxième solution et le solvant organique est compris entre 2:1 et 1:5, en particulier compris entre 1:0,9 et 1:1,1.

12. Méthode selon l'une quelconque des revendications 10 ou 11, dans laquelle le composé catalyseur est un sel organique, comme par exemple l'hexafluorophosphate de tétrabutylammonium, ou TBAPF6, à une concentration finale comprise entre 0,1 et 5 mM, en particulier entre 0,9 et 1,1 mM.

13. Méthode selon l'une quelconque des revendications 10 à 12, dans laquelle l'étape consistant à mélanger l'échantillon organique avec la solution finale est effectuée avec un rapport volumétrique entre la solution et l'échantillon compris entre 24:1 et 0,5:1, en particulier entre 1:0,95 et 1:1,05.

14. Méthode selon l'une quelconque des revendications précédentes, dans laquelle l'étape consistant à mélanger l'échantillon organique avec la solution finale est effectuée avec un rapport volumétrique entre la solution finale et l'échantillon compris entre 20:1 et 1,25:1, en particulier entre 4:0,95 et 4:1,05.
